# EUROPÄISCHE PATENTSCHRIFT

(11) **EP 0 936 905 B1**
(45) Veröffentlichungstag und Bekanntmachung des Hinweises auf die Patenterteilung: **27.08.2003**
(21) Anmeldenummer: 97911237.2
(22) Anmeldetag: 22.10.1997
(51) Int. Cl.: A61K 9/70

(54) **SOFORTIGE BENETZBARKEIT AUFWEISENDE(R) WASSERLÖSLICHER FILM ODER WASSERLÖSLICHE SCHICHT ZUR ORALEN APPLIKATION**
IMMEDIATE WETTABILITY WATER SOLUBLE FILM OR WATER SOLUBLE LAYER FOR ORAL APPLICATION
FILM HYDROSOLUBLE OU COUCHE HYDROSOLUBLE A MOUILLABILITE IMMEDIATE POUR APPLICATION ORALE

(30) Priorität: 11.11.1996 DE 19646392
(43) Veröffentlichungstag der Anmeldung: 25.08.1999
(60) Teilanmeldung: 03014213.7
(73) Patentinhaber: LTS LOHMANN Therapie-Systeme AG, 56626 Andernach (DE)
(72) Erfinder: ZERBE, Horst, Georg, Green Pond, NJ 07435 (US); SERINO, Anthony, J., Boonton, NJ 07005 (US); GUO, Jian-Hwa, Sparta, NJ 07871 (US)
(74) Vertreter: Flaccus, Rolf-Dieter, Dr.
(86) Internationale Anmeldenummer: EP9705820
(87) Internationale Veröffentlichungsnummer: WO98020862

(56) Entgegenhaltungen:
- EP-A- 0 250 187
- EP-A- 0 259 749
- EP-A- 0 781 550
- WO-A-91/05540
- WO-A-92/15289
- DE-A- 2 432 925
- US-A- 4 713 239
- US-A- 5 462 749

## Beschreibung

Die vorliegende Erfindung offenbart eine Zubereitung, die pharmazeutische Wirkstoffe und/oder die Atmung erfrischende Wirkstoffe enthalten, zur Anwendung in der Mundhöhle. Der Träger umfaßt wasserlösliche Polymere in Kombination mit bestimmten Inhaltsstoffen und hat eine therapeutische und/oder kosmetische Wirkung. Der Film wird unter Verwendung bestehender Beschichtungstechnologien aufgestrichen und getrocknet und weist eine sofortige Benetzbarkeit auf, gefolgt von schneller Auflösung/Zersetzung bei Applikation in der Mundhöhle.

An der Schleimhaut haftende (mukoadhäsive) Dosiersysteme zur Anwendung in der Mundhöhle, mit denen therapeutische und/oder kosmetische Wirkstoffe an die Mundschleimhaut abgegeben werden sollen, sind im Stand der Technik bekannt. Das US-Patent 5,047,244 beschreibt einen an der Mundschleimhaut haftenden Träger zur kontrollierten Abgabe eines therapeutischen Wirkstoffs durch das Schleimhautgewebe, der eine wasserfreie, aber hydratisierbare Polymermatrix und amorphes Siliciumdioxid enthält.
Fakultativ kann ein wasserunlöslicher Film beigefügt werden, um die Oberfläche nichtadhäsiv zu gestalten.
Die WO 91/06270 des gleichen Erfinders offenbart einen dreischichtigen Film zur verlängerten Abgabe eines aktiven Wirkstoffs in der Mundhöhle.
In gleicher Weise offenbart US 4,876,092 eine flächenförmige, adhäsive Zubereitung, umfassend eine adhäsive Schicht, welche bestimmte wasserlösliche und wasserunlösliche Polymere und einen wasserunlöslichen Träger enthält, welcher an der Mundschleimhaut haftet und dabei einen aktiven Wirkstoff an die Mundhöhle abgibt.

Alle diese vorgenannten Vorrichtungen sind nicht vollständig wasserlöslich und verbleiben in der Mundhöhle, selbst nach Erreichen des therapeutischen Ziels, und bereiten dem Patienten ein gewisses Unbehagen im Mund, das hauptsächlich durch die Trägerschicht verursacht wird, welche einen unlöslichen Rückstand im Mund zurückläßt.

Eine Reihe von Versuchen wurde unternommen, um das ungute Gefühl in der Mundhöhle zu verringern, welches durch die Starrheit und mangelnde Flexibilität der Trägerschicht verursacht wurde, indem man weiche Filmträger einführte. Die Dokumente EP 0 200 508 und EP 0 381 194 schlagen die Verwendung von Polyethylenfilmen, Polyvinylacetat, Ethylen-Vinylacetat-Copolymeren, Metallfolien, Laminaten aus Stoff oder Papier und einem Plastikfilm, und ähnlichen Materialien als weiche Träger vor, wobei synthetische Harze wie Polyethylen, Vinylacetat-Homopolymere und Ethylen-Vinyl-Acetat bevorzugte Materialien sind.
In gleicher Weise offenbart CA 1 263 312 die Verwendung von Polyolefinen wie Polyethylen, Polypropylen, Polyester, PVC, sowie Vliesstoffen als weiche Trägermaterialien.
Dennoch hinterlassen diese beim Patienten eine beträchtliche Menge von Rückständen des wasserunlöslichen Trägerfilms und verursachen daher immer noch ein unbehagliches Gefühl. Eine naheliegende Lösung zur Überwindung dieses Problems war die Entwicklung schleimhauthaftender Filme, welche vollständig zerfallen oder sich im Speichel auflösen.
Fuchs und Hilmann (DE 24 49 865.5) stellten homogene, wasserlösliche Filme für buccale Zuführung von Hormonen her. Sie schlugen die Verwendung von wasserlöslichen Cellulosederivaten wie Hydroxyethylcellulose, Hydroxypropylcellulose oder Methylhydroxypropylcellulose als Filmbildner vor.

Die beiden Patentschriften DE 36 30 603 und EP 0 219 762 offenbaren die Verwendung von quellbaren Polymeren wie Gelatine oder Maisstärke als Filmbildner, welche nach Applikation in der Mundhöhle langsam zerfallen, wobei sie einen aktiven Wirkstoff freisetzen, der im Film enthalten ist. Die gleichen Polymere können ebenfalls verwendet werden, um Filme herzustellen, die zur Zahnpflege dienen sollen, entsprechend der Beschreibung- in EP 0 452 446.
Aber auch diese Zubereitungen rufen ebenfalls ein ungutes Gefühl im Mund hervor, insbesondere verursacht durch ihre anfängliche Starrheit und verzögerte Erweichung. Hieraus resultiert ein Bedarf für eine Komposition zur Verwandung in der Mundhöhle, die. dem Erfordernis eines Wohlgefühls im Mund Rechnung trägt.
Die vorliegende Erfindung offenbart Verfahren und Zubereitungen, durch die ein unangenehmes Gefühl vermieden werden kann, und zwar durch Bereitstellen eines/einer sofortige Benetzbarkeit aufweisenden Films/Schicht zur Applikation an die Mundschleimhaut, und durch die eine Reißfestigkeit im freien Film erzielt wird, welche eine problemlose Beschichtung, Weiterverarbeitung und Verpackung eines verbraucherfreundlichen Produktes ermöglicht.

Die vorliegende Erfindung betrifft einen/eine schnellauflösende(n) Film/Schicht, der/die an der Mundhöhle haftet und dabei einen oder mehrere pharmazeutische oder kosmetische Wirkstoffe abgibt, wobei der Film/die Schicht ein oder mehrere wasserlösliche Polymere, einen oder mehrere Polyalkohole, eine Kombination von bestimmten Weichmachern oder oberflächenaktiven Stoffen und einen oder mehrere wirksame Inhaltsstoffe enthält. Wahlweise kann die Formulierung Farbstoffe, Süßmittel, Geschmacksstoffe, Geschmacksverbesserer oder andere Corrigenzien enhalten, wie sie üblicherweise zur Modifikation des Geschmacks von für die Applikation in der Mundhöhle bestimmten Formulierungen Verwendung finden. Der resultierende Film ist gekennzeichnet durch eine sofortige Benetzbarkeit, welche den Film unverzüglich nach Applikation an das Schleimhautgewebe erweichen läßt, wodurch beim Patienten ein länger anhaltendes unangenehmes Gefühl im Mund verhindert wird, sowie durch eine für normale Beschichtungs-, Schneid-, Aufschneid- und Verpackungsoperationen geeignete Reißfestigkeit.

Der mukoadhäsive Film der vorliegenden Erfindung enthält als wesentliche Bestandteile ein wasserlösliches Polymer oder eine Kombination von wasserlöslichen Polymeren, eine Mischung nichtionischer oberflächenaktiver Stoffe, ein oder mehrere Polyalkohole und einen oder mehrere pharmazeutische oder kosmetische Wirkstoffe.
Die für den mukoadhäsiven Film verwendeten Polymere umfassen hydrophile und/oder wasserdispergierbare Polymere. Bevorzugte Polymere sind wasserlösliche Cellulosederivate. Hydroxypropylmethylcellulose, Hydroxyethylcellulose und Hydroxypropylcellulose sind, allein oder gemischt, besonders bevorzugt. Beispiele für andere, fakultative, Polymere werden nachfolgend genannt, ohne die Erfindung einzuschränken: Polyvinylpyrrolidon, Carboxymethylcellulose, polyvinylalkohol, Natriumalginat, Polyethylenglycol, natürliche Gummen wie Xanthanharz, Traganth, Guarharz, Akazienharz, Gummiarabicum, wasserdispergierbare Polyacrylate wie Polyacrylsäure, Methylmethacrylatcopolymer, Carboxyvinylcopolymere. Die Konzentration des wasserlöslichen Polymers im fertigen Film kann zwischen 20 und 75 Gew.-% betragen. Eine bevorzugte Konzentration beträgt zwischen 50 und 75 Gew.-%.

Die für den mukoadhäsiven Film verwendete Mischung oberflächenaktiver Stoffe ist eine Mischung nichtionischer oberflächenaktiver Stoffe, die aus zwei Komponenten besteht. Die erste Komponente ist ein Polyethoxysorbitanfettsäureester oder ein α-hydro-ω-hydroxypoly(ethoxy)-poly-(propoxy)-poly(ethoxy)-Blockcopolymer, während die zweite Komponente ein Polyethoxyalkylether oder ein Polyethoxyrizinusölderivat ist.

Bevorzugt soll der HLB-Wert der Polyethoxysorbitanfettsäurester zwischen 10 und 20 betragen, wobei ein Bereich zwischen 13 und 17 besonders bevorzugt ist. Das α-hydro-ω-hydroxypoly(ethoxy)-poly(propoxy)-poly(ethoxy)-Blockcopolymer soll mindestens 35 Propoxy-Einheiten, bevorzugt mindestens 50 Propoxy-Einheiten enthalten.
Der Polyethoxyalkylether sollte einen HLB-Wert zwischen 10 und 20 besitzen, bevorzugt nicht weniger als 15. Das Polyethoxy-rizinusölderivat soll einen HLB-Wert zwischen 14 und 16 besitzen.
Um die erwünschte, sofortige Benetzbarkeit zu erreichen, wird das Verhältnis zwischen der ersten und der zweiten Komponente einer binären oberflächenaktiven Mischung zwischen 1:10 und 1:1 gehalten, bevorzugt zwischen 1:5 und 1:3.
Die Gesamtkonzentration der oberflächenaktiven Stoffe im fertigen Film hängt ab von den Eigenschaften der anderen Ingredienzien, soll jedoch üblicherweise zwischen 0.1 und 5 Gew.-% betragen.
Der Polyalkohol wird benötigt, um den erwünschten Weichheitsgrad des Filmes zu erreichen. Beispiele von Polyalkoholen umfassen Glycerin, Polyethylenglycol, Propylenglycol, Glycerinmonoester mit Fettsäuren, oder sonstige pharmazeutisch verwendete Polyalkohole. Die Konzentration von Polyalkohol in der Trockenmasse des Filmes beträgt üblicherweise 0.1 bis 5 Gew.-%.

Der Film ist besonders gut geeignet zur Abgabe eines weiten Bereiches pharmazeutisch aktiver Wirkstoffe durch die Schleimhautmeinbranen eines Patienten, insbesondere durch die buccalen Schleimhäute.
Therapeutische Wirkstoffe, die Absorptionsprobleme infolge begrenzter Löslichkeit, Abbau im Gastrointestinaltrakt oder extensiven Metabolismus haben, sind besonders gut geeignet.
Als Beispiele der einsetzbaren therapeutischen Wirkstoffe sind zu nennen: Hypnotica, Sedativa, Antiepileptica, Weckamine, Psychoneurotropica, Neuro-Muskelblooker, Antispasmodica, Antihistaminica, Antiallergica, Cardiotonica, Antiarrhythmica, Diuretica, Hypotensiva, Vasopressoren, Antitussiva, Expectorantia, Thyroidhormone, Sexualhormone, Antidiabetica, Antitumor-Wirkstoffe, Antibiotica sowie Chemotherapeutica und Narcotica. Die im Film einzulagernde Menge von Wirkstoff hängt von dessen Art ab und beträgt üblicherweise zwischen 0,01 und 20 Gew.-%, sie kann jedoch, falls zur Erzielung des gewünschten Effektes notwendig, höher liegen.

Kosmetische Wirkstoffe umfassen Atemerfrischer wie Menthol, andere Geschmacks-, Aroma- oder Duftstoffe, wie sie üblicherweise für die Mundhygiene verwendet werden, und/oder Wirkstoffe für Zahnpflege und/oder Mundhygiene, beispielsweise quartäre Ammoniumbasen. Die Wirkung von Geschmacksund Aromastoffen kann durch Geschmacksverstärker wie Weinsäure, Zitronensäure, Vanillin oder dergleichen verstärkt werden.
Farbstoffe, welche wahlweise dem Film beigemischt werden, müssen hinsichtlich Toxizität sicher sein und sollten zur Verwendung in Kosmetika durch die zuständigen Behörden zugelassen sein.

Der erfindungsgemäße mukoadhäsive Film kann folgendermaßen hergestellt werden:

Der Polyalkohol oder die Polyalkohole, oberflächenaktive Stoffe, Weichmacher und andere mögliche Bestandteile außer dem/den wasserlöslichen oder wasserdispersiblen Polymeren werden mit einer genügenden Menge eines kompatiblen Lösungsmittels gelöst. Beispiele eines kompatiblen Lösungsmittels umfassen Wasser, Alkohole oder deren Mischungen. Nach Bildung einer klaren Lösung wird das wasserdispersible Polymer oder die Mischung wasserdispersibler Polymere langsam unter Rühren und, falls notwendig, Hitze zugegeben, bis eine klare und homogene Lösung gebildet ist. Diese wird auf einen Träger aufgetragen und zu einem Film getrocknet. Das Trägermaterial muß eine Oberflächenspannung haben, die es ermöglicht, die Polymerlösung gleichmäßig über die vorgesehene Beschichtungsbreite zu verteilen, ohne daß die Lösung eingesaugt wird und somit eine destruktive Bindung zwischen Träger und Beschichtung entsteht.
Beispiele für geeignete Materialien umfassen nichtsilikonisierte Polyethylen-Terephthalat-Filme, nichtsiliconisiertes Kraftpapier, Polyethylen-imprägniertes Kraftpapier oder nichtsiliconisierten Polyethylenfilm.
Der Auftrag der Lösung auf das Trägermaterial kann mit jeder üblichen Vorrichtung ausgeführt werden. Eine speziell bevorzugte Auftragstechnik betrifft eine Walzenrakel-Streichmaschine.
Die Dicke der resultierenden Filmschicht hängt von der Konzentration der Feststoffe in der Beschichtungslösung sowie von der Spaltbreite der Beschichtungsmaschine ab und kann zwischen 5 und 200 µm variieren. Die Trocknung des Films wird in einem Heißluftbad unter Verwendung eines Trokkenofens, Trockentunnels, Vacuumtrockners oder anderer geeigneter Trockenvorrichtungen vorgenommen, welche die Wirkung des/der Wirkstoffs/Wirkstoffe oder der Geschmacksstoffe nicht negativ beeinträchtigen. Um ein unangenehmes Gefühl im Mund zuverlässig zu vermeiden, soll die Filmdicke 70 µm nicht überschreiten. Zur besseren Gebrauchserleichterung kann der Film in Stücke von geeigneter Größe und Form geschnitten und verpackt werden.

Die Erfindung wird anhand nachfolgender Beispiele veranschaulicht.

### Beispiel 1:

15 g Sorbit, 6 g Glycerin, 0,5 g Polysorbat 80 (Tween 80), 2 g Brij 35, 25 g Zitronenminzearoma, 3 g Aspartam, 15 g 1-Menthol und 3 g Zitronensäure werden bei 60°C in einer Mixtur von 250 g Wasser und 250 g Ethanol solange gerührt, bis sich eine klare Lösung gebildet hat.
Zu der Lösung werden 30 g Hydroxypropylmethylcellulose langsam unter Rühren zugegeben, bis eine klare und homogene Lösung gebildet ist. Die resultierende Lösung wird dann bis auf Raumtemperatur abkühlen gelassen und unter Verwendung einer üblichen Beschichtungs-/Trocknungsvorrichtung auf ein geeignetes Trägermaterial aufgestrichen, beispielsweise nichtsiliconisiertes polyethylenbeschichtetes Kraftpapier. Beschichtungsspalt und Bahngeschwindigkeit müssen so geregelt werden, daß eine Trockenfilmdicke zwischen 20 und 50 µm erreicht wird. Die Trockentemperatur hängt von der Länge des Trockenofens und der Materialgeschwindigkeit ab und soll so eingestellt werden, daß die Lösungsmittel vollständig, oder zumindest weitgehend vollständig vom Film entfernt werden. Der resultierende Film wird vom Träger abgelöst und zum Gebrauch in Stücke von geeigneter Größe und Form zerteilt.

### Beispiel 2:

3 g Sorbit, 1,5 g Kollidon 30 (Lieferant: BASF), 5 g Glycerin, 5 g Propylenglycol, 5 g Polyethylenglycol, 4 g Polysorbat 80 (Tween 80), 8 g Brij 35, 12 g Pfefferminzaroma, 0,8 g Aspartam werden in einer Mischung von 400 g Wasser und 400 g Ethanol bei 60°C unter Rühren aufgelöst. Zu der klaren Lösung werden 28 g Hydroxypropylmethylcellulose unter Rühren langsam zugegeben. Nach völliger Auflösung des Polymers wird die Lösung auf Raumtemperatur abgekühlt und auf einen Träger unter den gleichen Beschichtungs- und TRocknungsbedingungen wie in Beispiel 1 aufgestrichen. Der trockene Film wird wieder in Stücke von geeigneter Größe und Form zerteilt.

### Beispiel 3:

15 g Sorbit, 22,5 g Glycerin, 2,5 Propylenglycol, 2,5 g Brij 35, 2,5 g Poloxamer 407, 3,5 g Cremophor RH 40, 9 g Kräuterminzearoma, 0,5 g Aspartam werden unter ständigem Rühren bei 60°C in einer Mischung von 250 g Wasser und 250 g Ethanol gelöst. Zu der klaren Lösung werden 75 g Hydroxypropylcellulose unter ständigem Rühren langsam zugegeben. Mit der klaren Lösung wird wiederum beschichtet und unter den beschriebenen Bedingungen getrocknet, wie in Beispiel 1; der trockene Film wird in Stücke von geeigneter Größe und Form zerteilt.

## Patentansprüche

1. Zubereitung zur Anwendung in der Mundhöhle mit einem/einer an der Schleimhaut haftenden (mukoadhäsiven), sofortig benetzbaren Film oder Schicht, enthaltend eine homogene Mischung aus
- einem oder mehreren wasserlöslichen Polymer(en),
- einer Mischung nichtionischer oberflächenaktiver Stoffe,
- einem oder mehreren Polyalkohol(en),
- einem oder mehreren kosmetischen oder pharmazeutischen Wirkstoff(en), und gegebenenfalls
- einem oder mehreren Geschmacks- oder Aromastoff(en), **dadurch gekennzeichnet, dass** die Mischung der nichtionischen oberflächenaktiven Stoffe aus zwei Komponenten besteht, deren erste Komponente ein Polyethoxysorbitanfettsäureester oder ein α-hydro-ω-hydroxypoly(ethoxy)-poly(propoxy)-poly(ethoxy)-Blockcopolymer, und deren zweite Komponente ein Polyethoxyalkylether oder ein Polyethoxyrizinusölderivat ist, wobei das Verhältnis zwischen der ersten und der zweiten Komponente der binären oberflächenaktiven Mischung zwischen 1:10 und 1:1 gehalten wird.

2. Zubereitung nach Anspruch 1, **dadurch gekennzeichnet, dass** das Verhältnis zwischen der ersten und der zweiten Komponente der binären oberflächenaktiven Mischung zwischen 1:5 und 1:3 gehalten wird.

3. Zubereitung nach Anspruch 1 oder 2, **dadurch gekennzeichnet, dass** der HLB-Wert des Polyethoxysorbitan-fettsäuressters zwischen 10 und 20 beträgt.

4. Zubereitung nach einem oder mehreren der vorhergehenden Ansprüche, **dadurch gekennzeichnet, dass** der Polyethoxyalkylether einen HLB-Wert zwischen 10 und 20 besitzt.

5. Zubereitung nach einem oder mehreren der vorhergehenden Ansprüche, **dadurch gekennzeichnet, dass** das Polyethoxyrizinusölderivat einen HLB-Wert zwischen 14 und 16 besitzt.

6. Zubereitung nach einem oder mehreren der vorhergehenden Ansprüche, **dadurch gekennzeichnet, dass** das α-hydro-ω-hydroxypoly(ethoxy)-poly(propoxy)-poly(ethoxy)-Blockcopolymer mindestens 35 Propoxy-Einheiten enthält.

7. Zubereitung nach einem oder mehreren der vorhergehenden Ansprüche, **dadurch gekennzeichnet, dass** das wasserlösliche Polymer Hydroxypropylmethylcellulose, Hydroxyethylcellulose, Hydroxypropylcellulose, Polyvinylpyrrolidon, Carboxylmethylcellulose, Polyvinylalkohol, Natriumalginat, Polyethylenglycol, Xanthanharz, Traganth, Guarharz, Akazienharz, Gummiarabicum, Polyacrylsäure, Methylmethacrylatcopolymer, Carboxyvinylcopolymere oder deren Mischungen umfaßt.

8. Zubereitung nach einem oder mehreren der vorhergehenden Ansprüche, **dadurch gekennzeichnet, dass** der Polyalkohol ausgewählt ist aus der Gruppe, die aus Glycerin, Polyethylenglycol, Propylenglycol und Glycerinmonoestern mit Fettsäuren besteht.

9. Zubereitung nach einem oder mehreren der vorhergehenden Ansprüche, **dadurch gekennzeichnet, dass** der therapeutische Wirkstoff ausgewählt ist aus der Gruppe von Hypnotica, Sedativa, Antiepileptica, Weckaminen, Psychoneurotropica, Neuro-Muskelblockern, Antispasmodica, Antihistaminica, Antiallergica, Cardiotonica, Antiarrhythmica, Diuretica, Hypotensiva, Vasopressoren, Antiussiva, Expectorantia, Antitumor-Wirkstoffen, Thyroidhormonen, Sexualhormonen, Antidiabetica, Antibiotica sowie Chemtherapeutica und Narcotica.

10. Zubereitung nach Anspruch 1, **dadurch gekennzeichnet, dass** der kosmetische Wirkstoff ein Atemerfrischer wie Menthol, Geschmacks-, Aroma- oder Duftstoffe, wie sie üblicherweise für Mundhygiene verwendet werden, und/oder Wirkstoffe zur Zahn- und Mundpflege, wie quartäre Ammoniumbasen, umfasst.

## Claims

1. A composition applicable to the oral cavity comprising a film or sheet being adhesive to mucosa (mucoadhesive) with instant wettability, said film or sheet comprises a homogeneous mixture consisting of
- at least one water-soluble polymer,
- a mixture of nonionic surfactants,
- at least one polyalcohol,
- at least one cosmetically or pharmaceutically active agent, and
- optionally at least one flavor, **characterized in that**
the mixture of nonionic surfactants consists of two components, the first component being a polyoxyethylene sorbitan fatty acid ester or a α-hydro-ω-hydroxypoly(oxyethylene)poly(oxypropylene)poly(oxyethylene) block copolymer, and the second component being a polyoxyethylene alkyl ether or a polyoxyethylene castor oil derivative, wherein the ratio between the first and second component of the binary surfactant mixture is maintained between 1:10 and 1:1.

2. Composition according to claim 1, **characterized in that** the ratio between the first and second component of the binary mixture is between 1:5 and 1:3.

3. Composition according to claim 1 or 2, **characterized in that** the HLB value of the polyethoxyethylene sorbitan fatty acid ester is between 10 and 20.

4. Composition according to any one of the preceding claims, **characterized in that** the HLB value of the polyoxyethylene alkyl ether is between 10 and 20.

5. Composition according to any one of the preceding claims, **characterized in that** the polyoxyethylene castor oil derivative has an HLB value of 14 to 16.

6. Composition according to any one of the preceding claims, **characterized in that** the α-hydro-ω-hydroxypoly(oxyethylene)poly(oxypropylene)poly(oxyethylene) block copolymer contains at least 35 oxypropylene-units.

7. Composition according to any one of the preceding claims, **characterized in that** the water soluble polymer is selected from the group consisting of hydroxypropylmethyl cellulose, hydroxyethyl cellulose, hydroxypropyl cellulose, polyvinyl pyrrolidone, carboxymethyl cellulose, polyvinyl alcohol, sodium alginate, polyethylene glycol, xanthane gum, tragacantha, guar gum, acacia gum, arabic gum, polyacrylic acid, methylmethacrylate copolymer, carboxyvinyl compolymers, or mixtures thereof.

8. Composition according to any one of the preceding claims, **characterized in that** the polyalcohol is selected from the group consisting of glycerol, polyethylene glycol, propylene glycol, and glycerol monoesters with fatty acids.

9. Composition according to any one of the preceding claims, **characterized in that** the pharmaceutically active agent is selected from the group consisting of hypnotics, sedatives, antiepileptics, awakening agents, psychoneurotropic agents, neuromuscular blocking agents, antispasmodic agents, antihistaminics, antiallergics, cadiotonics, antiarrhythmics, diuretics, hypotensives, vasopressors, antitussive expectorants, thyroid hormones, sexual hormones, antidiabetics, antitumor agents, antibiotics, chemotherapeutics, and narcotics.

10. Composition according to claim 1, **characterized in that** the cosmetically active ingredient is selected from the group consisting of breath freshening compounds, flavors used for oral hygiene, fragrances used for oral hygiene, active agents used for oral cleansing, and active agents used for dental cleansing.

## Revendications

1. Préparation pour application dans la cavité buccale avec un film ou une couche mouillable immédiatement, adhérant à la muqueuse (mucoadhésif), contenant un mélange homogène constitué
- d'un ou plusieurs polymères hydrosolubles,
- d'un mélange de substances tensioactives non ioniques,
- d'un ou plusieurs polyalcools,
- d'une ou plusieurs substances actives cosmétiques ou pharmaceutiques et, le cas échéant,
- d'une ou plusieurs substances de saveur ou d'arôme,
**caractérisée en ce que** le mélange des substances tensioactives non ioniques est constitué de deux composants, le premier composant étant un ester d'acide gras de polyéthoxysorbitan ou un copolymère a-hydro-ω-hydroxypoly(éthoxy)-poly(propoxy)-poly(éthoxy) séquencé, et le deuxième composant étant un éther de polyéthoxyalkyle ou un dérivé d'huile de ricin polyéthoxylée, le rapport entre le premier et le deuxième composants du mélange tensioactif binaire étant maintenu entre 1 :10 et 1 :1.

2. Préparation selon la revendication 1, **caractérisée en ce que** le rapport entre le premier et le deuxième composants du mélange tensioactif binaire est maintenu entre 1 :5 et 1 :3.

3. Préparation selon la revendication 1 ou 2, **caractérisée en ce que** la valeur HLB de l'ester d'acide gras de polyéthoxysorbitan se trouve entre 10 et 20.

4. Préparation selon une ou plusieurs des revendications précédentes, **caractérisée en ce que** l'éther de polyéthoxyalkyle présente une valeur HLB entre 10 et 20.

5. Préparation selon une ou plusieurs des revendications précédentes, **caractérisée en ce que** le dérivé d'huile de ricin polyéthoxylée présente un valeur HLB entre 14 et 16.

6. Préparation selon une ou plusieurs des revendications précédentes, **caractérisée en ce que** le copolymère α-hydro-ω-hydroxypoly(éthoxy)-poly(propoxy)-poly(éthoxy) séquencé contient au moins 35 unités propoxy.

7. Préparation selon un ou plusieurs des revendications précédentes, **caractérisée en ce que** le polymère hydrosoluble comprend l'hydroxypropylméthylcellulose,l'hydroxyéthyl-cellulose,l'hydroxypropylcellulose, la polyvinylpyrrolidone, la carboxyméthylcellulose, l'alcool polyvinylique, l'alginate de sodium, le polyéthylèneglycol, la résine de xanthane, la gomme adragante, la résine de guar, la résine d'acacia, la gomme arabique, l'acide polyacrylique, un copolymère de méthacrylate de méthyle, des copolymères de carboxyvinyle, ou leurs mélanges.

8. Préparation selon une ou plusieurs des revendications précédentes, **caractérisée en ce que** le polyalcool est choisi dans le groupe constitué de glycérol, de polyéthylèneglycol, de propylèneglycol et de monoesters de glycérol avec des acides gras.

9. Préparation selon une ou plusieurs des revendications précédentes, **caractérisée en ce que** la substance active thérapeutique est choisie dans le groupe des hypnotiques, des sédatifs, des anti-épileptiques, des amines analeptiques, des psychoneurotropiques, des neuro-bloquants musculaires, des anti-spasmodiques, des anti-histaminiques, des anti-allergiques, des cardiotoniques, des anti-arythmiques, des diurétiques, des hypotenseurs, des vasopresseurs, des anti-tussifs, des expectorants, des substances actives anti-tumeurs, des hormones thyroïdiennes, des hormones sexuelles, des anti-diabétiques, des antibiotiques ainsi que des chimiothérapeutiques et des narcotiques.

10. Préparation selon la revendication 1, **caractérisée en ce que** la substance active cosmétique est un rafraîchisseur d'haleine comme le menthol, des matières de saveur aromatique, ou des parfums comme utilisés habituellement pour l'hygiène buccale, et/ou des substances actives pour les soins dentaires et de la bouche, comme les bases ammonium quaternaires.
